# EUROPÄISCHE PATENTSCHRIFT

(11) **EP 0 753 029 B1**
(45) Veröffentlichungstag und Bekanntmachung des Hinweises auf die Patenterteilung: **27.05.1998**
(21) Anmeldenummer: 95914287.8
(22) Anmeldetag: 21.03.1995
(51) Int. Cl.: C09C 3/08, C09C 3/04, C09D 17/00

(54) **DISPERGIER- UND MAHLHILFSMITTEL FÜR ANORGANISCHE FÜLLSTOFFE UND PIGMENTE UND IHRE VERWENDUNG**
DISPERSING AND GRINDING AUXILIARY FOR INORGANIC FILLERS AND PIGMENTS, AND USE THEREOF
AUXILIAIRES DE DISPERSION ET DE BROYAGE POUR CHARGES ET PIGMENTS INORGANIQUES ET LEUR UTILISATION

(30) Priorität: 26.03.1994 DE 4410662
(43) Veröffentlichungstag der Anmeldung: 15.01.1997
(73) Patentinhaber: Jungbunzlauer Ladenburg GmbH, D-68526 Ladenburg (DE)
(72) Erfinder: VON RAVEN, Axel, D-82402 Seeshaupt (DE)
(74) Vertreter: Grussdorf, Jürgen, Dr.
(86) Internationale Anmeldenummer: EP9501067
(87) Internationale Veröffentlichungsnummer: WO9526383

(56) Entgegenhaltungen:
- DE-A- 2 733 734
- US-A- 3 663 284
- US-A- 4 144 083
- US-A- 4 144 084
- US-A- 4 144 085

## Beschreibung

Die Erfindung betrifft Dispergier- und Mahlhilfsmittel für anorganische Füllstoffe und Pigmente, welche in der Lack-, Farben-, Keramik-, Gummi-, Kunststoff- und insbesondere in der Papierherstellung und -veredelung eingesetzt werden sollen.

Die Teilchengröße der Füllstoffe und Pigmente hat einen vielfältigen Einfluß auf die Eigenschaften der damit hergestellten Produkte, wobei überwiegend eine möglichst gleichmäßige Korngröße und Verteilung angestrebt wird. Eine Reagglomerisation unter Ausbildung ungleichmäßig großer Teilchen soll deshalb vermieden werden. Zu diesem Zweck ist es bekannt, beim Mahlprozeß oder vor der Weiterverarbeitung Dispergiermittel zuzusetzen, die eine Reagglomeration verhindern oder Agglomerate wieder in die Partikel zerteilen. Bei der Papierherstellung variieren die Eigenschaften, wie Weißgrad, Opazität, Glätte, Luftdurchlässigkeit und die Bedruckbarkeit sowie der Verschleiß von Papiermaschinensieben, Schneidmessern und Druckplatten stark mit der Teilchengröße der Pigmente und Füllstoffe. Trocken bzw. nicht dispergiert angelieferte Füllstoffe und Pigmente liegen je nach Art der Produkte in mehr oder weniger großen Agglomeraten vor. Die Teilchengröße der Streichpigmente beeinflußt sowohl das Viskositätsverhalten - oder allgemein das Laufverhalten - einer Streichfarbe auf der Streichanlage, wie Blade-Verschleiß, Rakelstreifen etc., als auch die Strichqualität hinsichtlich der optischen Eigenschaften und der Bedruckbarkeitseigenschaften (Weigl J. und J. Hlavatsch, 8. PTS-Symposium chemische Technologie der Papierherstellung, München, 1988, Seite 19-28).

Bei Streichpigmenten, die im trockenen Zustand angeliefert werden, liegen die Primärteilchen - je nach Teilchengröße und spezifischer Oberfläche - als Agglomerate bzw. grobteilige Aggregate vor. Diese Agglomerate wirken sich bekanntlich sowohl auf die Verarbeitung der Streichfarbe als auch auf die Strichqualität nachteilig aus. Für einen optimalen Einsatz der Füllstoff - und Streichpigmentteilchen bei der Papierherstellung und -veredelung ist die Voraussetzung, daß die einzelnen Pigmente als Primärteilchen, d. h. entflockt, vorliegen. Zur Dispergierung dieser Agglomerate und zur Erzielung hoher Feststoffgehalte ist die kombinierte Anwendung von geeigneten Dispergiergeräten und der Einsatz von chemischen Hilfsmitteln erforderlich. Eine optimale Dispergierung stellt sicher, daß Anziehungskräfte zwischen den Teilchen eliminiert werden oder diesen entgegengewirkt wird und so eine Reagglomeration der Pigmentteilchen nach der Herstellung der Dispersion verhindert wird. Dies ist vor allem für die Slurry-Lieferung bzw. Slurry-Lagerung sehr wichtig.

Bei den gebräuchlichsten anorganischen und organischen Dispergiermitteln, die in der Papierindustrie eingesetzt werden, handelt es sich fast ausschließlich um Polyanionen, vorzugsweise Polyphosphat oder Polyacrylat, die folgendes bewirken sollen (Weigl J., von Raven A. und Hofmann H.P. Neue Dispergiersysteme - Auswirkungen auf die Streichfarbe und die Wiederverarbeitung des Streichausschusses, Wochenblatt für Papierfabrikation 117, 1063-1070 (1989) Nr. 23/24).
- Dispergierung der Pigmentagglomerate zu Primärteilchen,
- Verbesserung der optischen Eigenschaften bei gestrichenen Papieren,
- Erzielung hoher Feststoffgehalte,
- Erhaltung der optimalen Deflokulation bei erhöhter Lager- und Verarbeitungstemperatur
- Vermeidung von starken Viskositätserhöhungen durch Wasserverlust bei der Rezirkulation der Streichfarbe
- Erzielung recyclinggerechter Produkteigenschaften
- ökologische Unbedenklichkeit

Der Enfflockungsmechanismus mit negativ geladenen Dispergiermitteln dürfte so vor sich gehen, daß das Dispergiermittel an der Oberfläche adsorbiert wird und positiv geladene Pigmente - wie z.B. Aluminiumoxide, Satinweiß, evtl. Titandioxid und Kreide - um lädt bzw. bei anionischen Pigmenten eine beträchliche Zunahme des negativen Zeta-Potentials bewirkt. Es hat sich gezeigt, daß als wichtigste Eigenschaft für die Dispergierung eine möglichst große Anzahl an negativen Ladungen pro Molekül wichtig ist und daß daneben eine gute Adsorption an den jeweiligen Teilchen gewährleistet sein muß (Rohmann M.E., Die Wirkungsweise der Dispergiermittel in der Papierstreicherei, Wochenblatt für Papierfabrikation 101, 79-87 (1973) Nr. 3.

Die gebräuchlichen Dispergiermittel unterteilt man im allgemeinen in solche anorganischen und solche organischen Ursprungs. Sieht man von starken Basen wie Natronlauge und Ammoniak ab, die unter gewissen Bedingungen eine dispergierende Wirkung ausüben werden überwiegend Polyanionen eingesetzt. Für diese Zwecke sind organische und anorganische Dispergiermittel bekannt. Als anorganische Dispergiermittel werden z.B. Phosphate und Polyphosphate, wie Natriumhexametaphosphat, eingesetzt. Sie zeigen eine gute Dispergierwirkung, haben aber den Nachteil, daß sie nicht hydrolysestabil sind. Dies bedeutet, daß solche Dispersionen bei längerer Lagerung sich in unerwünschter Weise verdicken. Von den organischen Produkten wird Natriumcitrat u. a. als Dispergiermittel für Füllstoffe und Streichpigmente erwähnt, das jedoch keine besonders gute Dispergierwirkung aufweist (Rohmann M.E., Die Wirkungsweise der Dispergiermittel in der Papierstreicherei, Wochenblatt für Papierfabrikation 101, 79-87 (1973) Nr. 3; Hagemeyer R. W., A Project of the Coating Pigments Committee TAPPI Monograph Series Nr. 38).

Dagegen fand Lapin (Lapin, V. V. EUCEPA SYMP. Chemistry in Papermaking (Warschau) Sept. 1978, Seite 80-89) annähernd gleiche Stabilitätseigenschaften bei der Dispergierung von Kaolin mit Polyacrylat und Natriumcitrat, jedoch bei wesentlich höheren Zugabemengen von Natriumcitrat. Während Trinatriumcitrat als Builder in der Wasch - und Reinigungsmittel zunehmend an Bedeutung gewinnt, konnten sich diese Produkte in der Papierindustrie bisher nicht durchsetzen.

Im US- Patent 4.144.085 wird darauf hingewiesen, daß durch geringe Zugabemengen von Natrium-Gluconat zu dispergierten Kaolin-Slurries die Lagerstabilität positiv beeinflußt wird. Ein Einfluß auf die primäre Dispergierung wurde dabei nicht gefunden, da die Ausgangsviskosität der Slurries unabhängig vom Zusatz der Gluconate ist.

Aus der US-P 4,144,083 ist bekannt, daß auch Citronensäure und ihre Salze in der Lage sind, bei nachträglicher Zugabe Kaolin-Slurries, die bereits suspendiert sind, bei der Lagerung zu stabilisieren. US-P 4,144,083 nennt dazu Weinsäure und ihre Salze.

Aus der US-P 3 663 284 ist bekannt, daß Citronensäure oder Weinsäure oder ihre Salze in der Lage sind, Titandioxidpigment-Slurries gegen Sedimentation zu stabilisieren und auch eingefrorene Sedimente wieder aufzutauen. Carrageen, Carboxymethylcellulose oder andere Polymere werden als zusätzliche Dispersionsmittel vorzugsweise zugegeben, um die Stabilität zu verbessern.

In der Papierindustrie werden als Dispergiermittel vorwiegend Polymere und Copolymere der Methacryl- und Acrylsäure mit niedrigem bis mittlerem Molekulargewicht eingesetzt. Insbesondere für die Dispergierung von Talkum werden außer Polyacrylaten auch Tenside als Netzmittel und Schutzkolloide z.B. Carboxymethylcellulose (CMC) verwendet.

Der Einsatz von anionischen Dispergiermitteln oder auch von anionischen Mahlhilfsmitteln auf Polyacrylat-Basis, wie sie z. B. bei der CaCO₃ Mahlung verwendet werden, kann neben den oben genannten positiven Wirkungen auch mit Nachteilen verbunden sein:
- Verringerung der Bindemitteladsorption am Pigment durch hohe gleichnamige Aufladung( Abstoßungspotentiale),
- verstärkte Bindemittelpenetration und -migration,
- Erniedrigung des Sedimentvolumens,
- Produktionsbeeinträchtigung durch ungewollte Wechselwirkungen mit kationischen Hilfsmitteln.

Den negativen Auswirkungen von anionischen Dispergiermitteln auf die ersten drei genannten Punkte wird heute durch eine gewisse Ladungsreduzierung mit entsprechenden Bindersystemen (z.B. carboxylgruppenreiche Binder) und durch den Zusatz von niedermolekularen kationischen Hilfsmitteln, sogenannten Immobilisierungsmittel sowie durch entsprechende verfahrenstechnische Maßnahmen mehr oder weniger entgegengewirkt.

Als immer problematischer erweist sich jedoch der letzte Punkt, die Produktionsbeeinträchtigung durch verschiedene anionische Dispergiermittel, vor allem auf Polyphosphat- und Polyacrylat-Basis, die z. B. über die Streichausschußverarbeitung bzw. die Streichfarbenrückführung in den Papierherstellungsprozeß gelangen. Besonders bei stark eingeengten Wasserkreisläufen und der neutralen Papierherstellung, bei der die Al⁺³-lonen als Fällungs- bzw. Ladungsneutralisationsmittel fehlen, treten diese anionischen Dispergiermittel in ungewollte Wechselwirkung mit kationischen Hilfsmitteln und wirken dadurch als sogenannte "Störstoffe". Diese Wechselwirkung zwischen den kationischen Hilfsmitteln und den anionischen Dispergiermitteln führt durch Komplex-Koazervation zu einer starken Wirksamkeitsbeeinflussung dieser Hilfsmittel sowie zu Ablagerung und somit zu erheblichen Produktionsstörungen. Es ist bekannt, daß allein zur Neutralisation von anionischen Dispergiermitteln auf Polyacrylat-Basis im Jahr bis zu 5 Mio. DM an kationischen Hilfsmitteln eingesetzt werden müssen (Weigl J., von Raven A. und Hofmann H.P., Neue Dispergiersysteme - Auswirkungen auf die Streichfarbe und die Wiederverarbeitung des Streichausschusses).

Wie bereits erwähnt, hat die Teilchengröße der Füllstoffe einen vielfältigen Einfluß auf die Qualitätseigenschaften des Papiers wie z.B. Opazität, Glätte, Luftdurchlässigkeit und vor allem auf die Bedruckbarkeit. Wegen der erwähnten Störstoffprobleme wird in den meisten Fällen auf die Zugabe von Dispergiermitteln beim Filterstofffeinsatz bei der Papierherstellung verzichtet. Die heute zum größten Teil angewandte Dispergierung mit Natronlauge führt nur zu einem unzureichenden Dispergiereffekt und zu anderen, durch die Extraktion im alkalischen pH-Bereich, zu höheren "Störstoffen" und zu verstärkten Ablagerungen.

Es stellte sich daher die Aufgabe, Dispergier- und Mahlhilfmittel zu finden, die diese Nachteile nicht oder vermindert aufweisen.

Die Lösung dieser Aufgabe wird durch die Merkmale des Hauptanspruchs gegeben und durch die der Unteransprüche gefördert.

Überraschenderweise wurde nun gefunden, daß durch gezielte Einstellung der lonenkonzentration mit einer Mischung aus Citronensäure und Gluconsäure sowie ihren Alkalisalzen, wobei Citrat und Gluconat in einem Verhältnis von 1 : 20 bis 20 : 1 vorliegen, ein wirksames Dispergiermittel erhalten wird. Durch einen synergistischen Effekt sind über einen weiten pH-Bereich höhere Feststoffgehalte bzw. niedrigere Viskositätswerte sowie höhere Stabilitätseigenschaften bei der Lagerung der dispergierten Füllstoff- und Pigmentdispersionen erzielbar als mit handelsüblichen Produkten, ohne deren Nachteile wie "Störstoffwirkung" hinnehmen zu müssen. PH-Bereiche der Dispersion von 4,0 - 10,5, vorzugsweise 8,3 - 9,0 bzw. 6,0 - 6,8, sind brauchbar. Der pH-Wert wird durch Zugabe von Alkalihydroxid oder -carbonat bzw. durch die entsprechenden Säuren eingestellt. Die Dispergiermittel werden dabei in einer Menge von 0,05 - 2 Gew.%, vorzugsweise 0,1 - 1 Gew.%, bezogen auf die Feststoffe, verwendet.

Sowohl bei TNC, Na-Gluconat, Citronensäure und Gluconsäure als auch bei deren Kombinationen treten keine ungewollten Wechselwirkungen mit kationischen Hilfsmitteln auf, d. h. das neue Dispergiermittelsystem wirkt nicht als "Störstoff" bei der Papierherstellung. Somit lassen sich Füllstoffe optimal dispergieren, mit verbesserter Papier- und Druckqualität, ohne Produktionsprobleme und Mehrverbrauch an kationischen Hilfsmitteln. Bevorzugt wird eine Mischung aus Citrat und Gluconat im Verhältnis 1 : 2 bis 5 : 1.

Weiterhin bevorzugt ist es, der Mischung zusätzlich eine oder mehrere Hydroxycarbonsäuren mit 3 bis 10 C-Atomen , welche 1 bis 6 Carbonsäuregruppen und 1 bis 5 OH-Gruppen enthalten, insbesondere Weinsäure, Äpfelsäure, Glucarsäure, Glucuronsäure oder ihre Natrium- und/oder Kaliumsalze, zuzufügen.

Während die heute vorwiegend in der Papierindustrie verwendeten ionischen Dispergiermittel auf Polyacrylat- und z. T. auf Polyphosphat-Basis die Stabilisierung hauptsächlich über das gegenseitige hohe Abstoßungspotential (hohes Zeta-Potential) erfolgt, ergibt aufgrund des unterschiedlichen Wirkungsmechanismus des neuentwickelten Dispergiersystems eine weit geringere Ladungsdichte bzw. Zeta-Potential an der Pigmentgrenzfläche.

Bei dem neuen Dispergiersystem dürfte es sich um eine Überlagerung von elektrostatischen, sterischer und Schutzkolloid- Wechselwirkungen mit ausgezeichneten Komplexbildungseigenschaften gegenüber Kationen handeln. Daraus ergeben sich bei der Herstellung von Streichfarben gegenüber den handelsüblichen polyionischen Dispergiermittel einige Vorteile:

Mit abnehmendem Zeta-Potential der Streichfarbe ist in der Regel eine Zunahme des relativen Sedimentvolumens (RSV) und somit ein höheres Strichvolumen und bessere Faserabdeckung verbunden.

Eine zu hohe gleichnamige Aufladung von Pigment, Binder, Cobinder und sonstigen Additiven wirkt einer Adsorption dieser Stoffe an der Pigmentoberfläche entgegen. Dadurch liegen diese Komponenten frei in der fluiden Phase der Streichphase vor und können nach deren Kontakt mit der trockenen Papieroberfläche zusammen mit dem Wasser leicht in das Basispapier penetrieren, womit eine Verarmung an diesen Stoffen in der Strichschicht einhergeht.

Desweiteren ist bekannt, daß stark anionische, nicht adsorbierte, gelöste Binder bzw. Cobinder, wie z.b. Carboxymethycellulose (CMC) oder Stärke, bei der Trocknung besonders stark zu einer Bindemittelmigration an die Strichoberfläche neigen. Die Folgen, wie Glanzeinbußen oder Bedruckbarkeitsbeeinträchtigungen in Form von z. B. Mottling, sind hinlänglich bekannt. (Engström G., Fineman I., Perrson A., Karlson I. und Akesson I. - Trocknung von stärkehaltigen Streichfarben - Erfahrungen mit der Trocknung in Schwebetrocknern, Wochenblatt für Papierfabrikation 108, 793-797 (1980) Nr. 16).

Durch die bessere Wirksamkeit sowie aufgrund des niedrigen Zeta-Potential des neuen Dispergiersystems und der dadurch besseren Adsorption der Binde- und Hilfsmittel auf der Pigmentoberfläche kann die Zugabemenge an wasserlöslichen Hilfsmitteln zur Streichfarbe insgesamt eventuell verringert und somit die Qualität der erzeugten Produkte v. a. hinsichtlich der Be- und Verdruckbarkeitseigenschaften gesteigert werden.

Weiterhin wird, wie bereits erwähnt, durch das neue Dispergiersystem die Streichfarben-rückführung , die Ausschußverarbeitung und der Altpapiereinsatz durch die Reduzierung der Störstoff-Fracht erleichtert, d.h. schon bei der Herstellung des Papiers wird dessen Wiederverarbeitung berücksichtigt ("Recyclinggerechtes Produkt").

Citronensäure und ihre Salze sind in der belebten Natur außerordentlich weit verbreitet. Neben ihrem Vorkommen in zahlreichen zum menschlichen Verzehr geeigneten Pflanzen und Früchten ist Citronensäure ein zentrales Zwischenprodukt beim Abbau von Nahrungsmitteln zu Kohlendioxid und Wasser. Bei einem ubiquitären Stoffwechselprodukt ist die biologische Abbaubarkeit naturgemäß gut. Citronensäure und Trinatriumcitrat werden im OECD-Test innerhalb von 24 Stunden zu 98 % abgebaut. Die Abwasserbehandlung wird nicht negativ beeinflußt. Citronensäure weist grundsätzlich eine geringe, überwiegend pH-bedingte akute aquatische Toxizität auf. Citrate wirken nicht eutrophierend. Feste Citronensäure und Citronensäurelösungen unter einem Gehalt von 50% sind in die Wassergefährdungsklasse 0 eingestuft. Das neutrale TNC hat gegenüber Gewässer ein noch niedrigeres Gefährdungspotential. Allgemein kann man sagen, daß nicht zuletzt Umweltgesichtspunkte die Citrate als Ersatz für TPP ins Blickfeld gerückt haben.

Entsprechend gut abbaubar sind auch Natriumgluconat und D-Gluconsäure (2,3,4,5,6,-Pentahydroxyhexan-1-carbonsäure), die fementativ aus Glucose (Dextrose) gewonnen wird. D-Gluconsäure ist nicht toxisch, gut biologisch abbaubar und verursacht keine Abwasserprobleme. Natriumgluconat bildet stabile Komplexe mit mehrwertigen Metallen, insbesondere in alkalischen Lösungen, wo andere Komplexbildner in ihrer Wirkung stark nachlassen, wodurch der viskositätserhöhenden bzw. flockenden Wirkung erfolgreich entgegengewirkt werden kann.

Das Gleiche gilt für andere durch Oxidation von Zuckern gewonnene Säure, wie Glucuronsäure oder Glucarsäure, sowie andere in der Natur vorkommende Hydroxycarbonsäuren wie Weinsäure oder Äpfelsäure und ihre Salze.

Die üblichen Dispergiermittel wie Polyacrylate sind die Salze von relativ schwachen Säuren und starken Basen, in den meisten Fällen Natronlauge. Damit diese Produkte ihre maximale Dispergierwirkung enffalten können, müssen alle sauren Gruppen in der anionischen Form vorliegen, d.h. völlig neutralisiert sein. Wenn man nun z. B. Polyacrylsäure mit Natronlauge titriert, erreicht man den Äquivalenzpunkt, je nach der Konzentration der Lösung, erst zwischen pH 8 und 9, in diesem Gebiet ist auch das Maximum der Dispergierung. Es ist ferner bekannt, daß bei weiterer pH-Wert-Steigerung die Viskosität der Clayanschlämmung (Tonerde) wieder zunimmt. Dies ist wahrscheinlich darauf zurückzuführen, daß, wie Bidwell, Jepson und Toms nachgewiesen haben, mit steigendem pH-Wert die Adsorption von Polyacrylat am Clay geringer wird, da die wachsende Menge an OH⁻-lonen mit dem Dispergiermittel in Wettstreit um die zu besetzenden Stellen am Clay tritt. Durch das wachsende Angebot an Hydroxylionen wird das gut dispergierende Polyanion durch das wesenlich schwächere Hydroxylion ersetzt. Hinzu kommt, daß mit steigender NaOH-Zugabe natürlich auch die Salzkonzentration in der flüssigen Phase beträchtlich erhöht wird, wodurch ebenfalls ein Viskositätsanstieg auftritt (Rohmann M.E., Die Wirkungsweise der Dispergiermittel in der Papierstreicherei, Wochenblatt für Papierfabrikation 108, 793-797 (1980) Nr. 16; Hagemeyer R. W., A Project of the Coating Pigments Committee TAPPI Monograph Series Nr. 38).

Neben dem kombinierten Stabilisierungsmechanismus des neuen Dispergiersystems "Abstoßungspotential", "sterischer Stabilisierung" und "Schutzkolloidwirkung", sowie Komplexierung von mehrwertigen Kationen, besteht die Möglichkeit durch Zugabe von NaOH aus Citronensäure, Weinsäure, Äpfelsäure etc. neutralisierte bzw. teilneutralisierte Produkte über einem weiten pH-Wirkungsbereich, ohne zu hohe Salzkonzentration, mit ausgezeichneter Dispergierwirkung herzustellen.

Während man mit TNC mit relativ geringen NaOH-Zugabemengen gute Dispergiereffekte erzielt, müssen mit zunehmenden Natriumgluconat-Anteilen erhöhte Mengen NaOH dosiert werden. Bei einer Dispergierung mit reinem Na-Gluconat muß etwas mehr NaOH vorgelegt werden, bzw. im Laufe der Dispergierung ergänzt werden.

Je nach Füllstoff- bzw. Pigmentart sowie dem gewünschten pH-Wert des Pigmentes oder der Streichfarbe erfolgt dann die Citronensäure bzw. Weinsäuredosierung.

Es lassen sich selbstverständlich fertige Dispergiermittelgemische, deren Zusammensetzung von dem jeweiligen Einsatzgebiet abhängen, einsetzen. Das erfindungsgemäße Dispergiersystem läßt sich selbstverständlich auch mit anderen Dispergiermittel anwenden wie z.B. bei der Nachdispergierung eines mit Polyacrylat gemahlenen Calciumcarbonats.

Die in diesem Verfahren einsetzbaren anorganischen Füllstoffe oder Pigmente sind z. B. Aluminiumsilikate (z.B. Kaolin), Magnesiumsilikate (z. B. Talkum), präz. Calciumcarbonate, natürlich gemahlene Calciumcarbonate (z. B. Kreide, Kalkstein, Marmor, Calcit, Aragonit), Calciumsulfat, Glimmer, Titandioxid, Aluminiumhydroxid, Satinweiß, Bentonit, sowie deren Mischungen.

Mit den erfindungsgemäßen Mitteln lassen sich Dispersionen von Füllstoffen, Pigmenten und deren Mischungen besonders vorteilhaft herstellen, die bei der Lack-, Farben-, Keramiken-, Gummi-, Kunststoff- und insbesondere der Papierherstellung und -veredelung angewendet werden.

Die folgenden Beispiele erläutern die Erfindung ohne sie darauf zu beschränken.

Der Viskositätsmessung als Maß für den Dispergierungsgrad liegt die Überlegung zugrunde, daß mit steigender Dispergierung zwar die Oberfläche des Pigmentes vergrößert wird, die Dicke der sie umgebenden Wasserschicht jedoch verringert wird und die Tendenz zu Ausbildung einer Struktur abnimmt, wodurch allgemein die Beweglichkeit der Teilchen vergrößert wird. Die Dispergiermittelmenge richtet sich nach der Art des Pigments, spez. Oberfläche, Polarität, Teilchengröße und - form, dem Feststoffgehalt, pH-Wert und danach, ob oder wieviel eines solchen Mittels eventuell vom Pigmenthersteller zugesetzt wurde. Die für die Minimalviskosität erforderliche Menge an Entflockungsmittel erhält man durch Auftragen der Viskosität gegen den Entflockungsmittelzusatz.

Die Dispersionen wurden auf 20 °C temperiert und dann pH-Wert und Viskosität gemessen.

Das Dispersionsmedium ist üblicherweise reines Wasser, jedoch können gewünschtenfalls auch polare organische Lösungsmittel, wie Alkohol oder Netzmittel zugesetzt werden.

Die Viskositätsmessung erfolgte auf einem Brookfield Viskosimeter Modell RVT. Bei den Standarddrehzahlen von 50 U/min und 100 U/min wird ein Geschwindigkeitsgefälle von ca. 61 s⁻¹ und 122 s⁻¹ aufgebracht.

Wenn der Dispergiermittelbedarf sowie ggf. die notwendige NaOH- oder Säuremenge bekannt war, wurde nach folgender Arbeitsweise vorgegangen:
- Wasser vorlegen
- Dispergiermittel zugeben
- Rührer einschalten
- Pigment eintragen (nicht zu schnell !), es dürfen sich keine Taschen mit trockenem Material am Boden oder an der Wand bilden
- Rührer je nach Pigment 5-30 min so laufen lassen, daß keine Luft eingezogen wird

Wenn die Dispergiermittel und NaOH- bzw. Säurezugaben variiert werden sollen, wurde wie folgt vorgegangen:
- Wasser vorlegen
- Rührer einschalten
- Pigment eintragen bis Viskosität im Bereich 300-1000 mPas liegt
- vorgelöstes Dispergiermittel (Verdünnung nicht zu hoch) tropfenweise zugeben, bis der Tropfen keine Viskositätsabnahme mehr bewirkt
- erneut Pigment eintragen (wie oben)
- erneut Dispergiermittel bzw. NaOH tropfenweise zugeben (wie oben)
- Wasser-, Pigment- und Dispergiermittelmenge notieren.

Besondere Arbeitsweise für Talkum, Ruß und andere hydrophobe Stoffe:
- Wasser vorlegen
- Strömungsbecher wegklappen
- Netzmittel zugeben (ca. 0,1-0,5 % atro)
- Dispergiermittel zugeben
- Rührer langsam laufen lassen
- Talkum langsam und gleichmäßig eintragen
- Strömungsbecher einklappen
- Rührgeschwindigkeit erhöhen
- Entschäumer zugeben
- 15 -30 min rühren

Folgende Abkürzungen werden verwendet:
otro = ofentrocken
atro = auf Trockensubstanz
lutro = lufttrocken

### Beispiel 1

Dem in einem 1000 ml Becherglas vorgelegten, benötigtem Wasser wurde das Dispergiermittel zugegeben und anschließend etwa eine Minute gemischt. Danach wurden 400 g otro Kaolin in kleinen Portionen zugegeben und anschließend 15 min bei 1400 U/min mit einer 80 mm Dissolverscheibe dispergiert. Der pH-Wert wurde nach diesem Dispergieren eingestellt. Das Zugeben von 0,7 ml NaOH (50%ig) zeigte bei den Versuchen Polyacrylat, TNC und 85 % TNC und 15 % Na-Gluconat keine offensichtliche Wirkung. Bei den Versuchen 4, 5 und 6 erniedrigte sich die Viskosität sichtlich bei der Zugabe von NaOH. Bei Versuch 7 war ohne die Zugabe von NaOH keine Dispergierung möglich. Die Dispergierwirkung der Natronlauge ist bei Versuch 8 am deutlichsten. Zum Einstellen des pH-Wertes wurde bei Versuch 8 weitere 0,2 ml NaOH zugegeben. Alle Dispersionen wurden auf 20 °C temperiert und dann pH-Wert und Viskosität gemessen.

### Beispiel 2

Es wurde wie im Beispiel 1 beschrieben verfahren, jedoch statt 0,7 ml NaOH wurden nun 0,3 ml NaOH kurz vor Ende des letzten Pigmenteintrages zugegeben.

### Beispiel 3

In einem 1000 ml Becherglas wurde die für einen bestimmten Feststoffgehalt und 400 gr otro Pigment erforderliche Menge Wasser und das jeweilige Dispergiermittel, bzw. 0,20 ml NaOH bei dem Versuch mit NaOH, vorgelegt. Bei den anderen Versuchen wurde kein NaOH eingesetzt. Nach etwa 30 sec dauerndem Mischen wurde die berechnete lutro Pigmentmenge in kleinen Portionen bei einer Drehzahl von 500 U/min des Laborrührers, bestückt mit einer 8 mm Dissolverscheibe, eingestreut. Nach Beendigung des Einstreuens wurde der pH-Wert eingestellt und anschließend 15 min bei 1400 U/min dispegiert. Alle Proben wurden auf 20 °C temperiert und dann der pH-Wert und die Viskosität gemessen nach Brookfield bei 50 und 100 U/min geprüft.

### Beispiel 4

Es wurde wie in Beispiel 3 beschrieben verfahren, jedoch wurde bei dieser Versuchsreihe 1,1 ml NaOH zugegeben.

### Beispiel 5

Es wurde wie in Beispiel 4 beschrieben verfahren, mit Ausnahme von Talkum. Bei der Talkum-Dispergierung wurde wie beschrieben verfahren.

### Beispiel 6

Mit der Polyelektrolyt-Titration wird der Verbrauch von kationischen Poly-DAD-MAC (Polydiallyldimethylammoniumchlorid) bestimmt, der für die Ladungsneutralisation anionische Dispergiermittel benötigt wird.

Die Versuchsdurchführung erfolgten gemäß der Vorschrift "Bestimmung der Ladungsdichte von ionischen Polyelektrolyten mittels Polyelektrolyt-Titration", PTS-Methode, PTS-Verlag, München, 1993.

### Beispiel 7

Unter den konventionellen Methoden erlaubt die Mikroelektrophorese die schnellste Bestimmung des Zeta-Potentials. Die Untersuchungen wurden mit dem Mobility -Meter Mark 1 durchgeführt. Die Mobilitätsmessung erfolgte durch visuelle Beobachtung der Teilchenbewegung über ein Mikroskop. Die Mobilität kann digital abgelesen werden. Aus der gemessenen Mobilität läßt sich dann das Zeta-Potential (ZP) berechnen. Die Konzentration der untersuchten Pigmentdispersion betrug 0,5%.

Die Versuchsdurchführung erfolgte gemäß der Vorschrift "Bestimmung des Zeta-Potentials von ionischen Polyelektrolyten mittels Polyelektrolyt-Titration" PTS-Methode, PTS-Verlag, München, 1993.

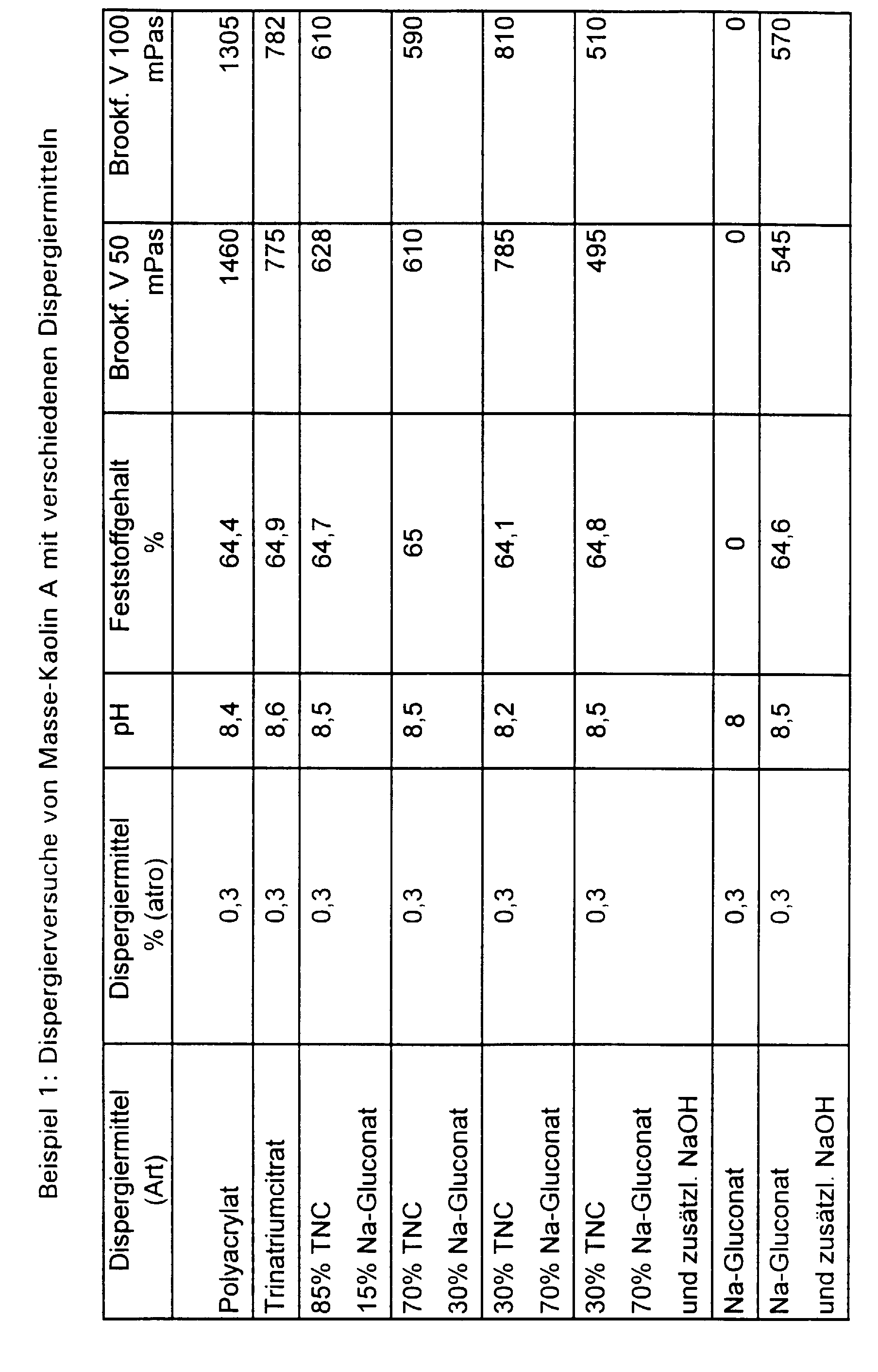

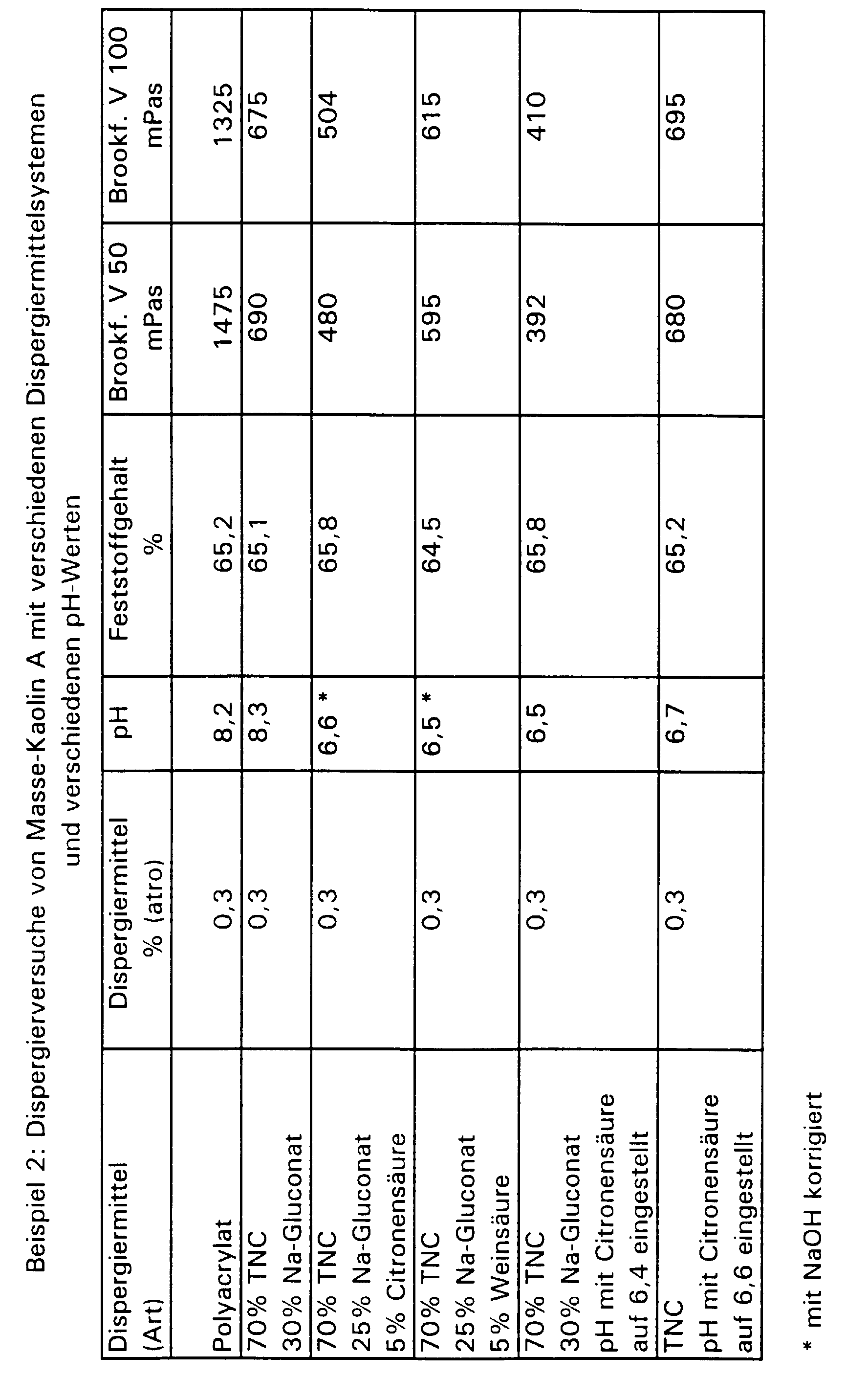

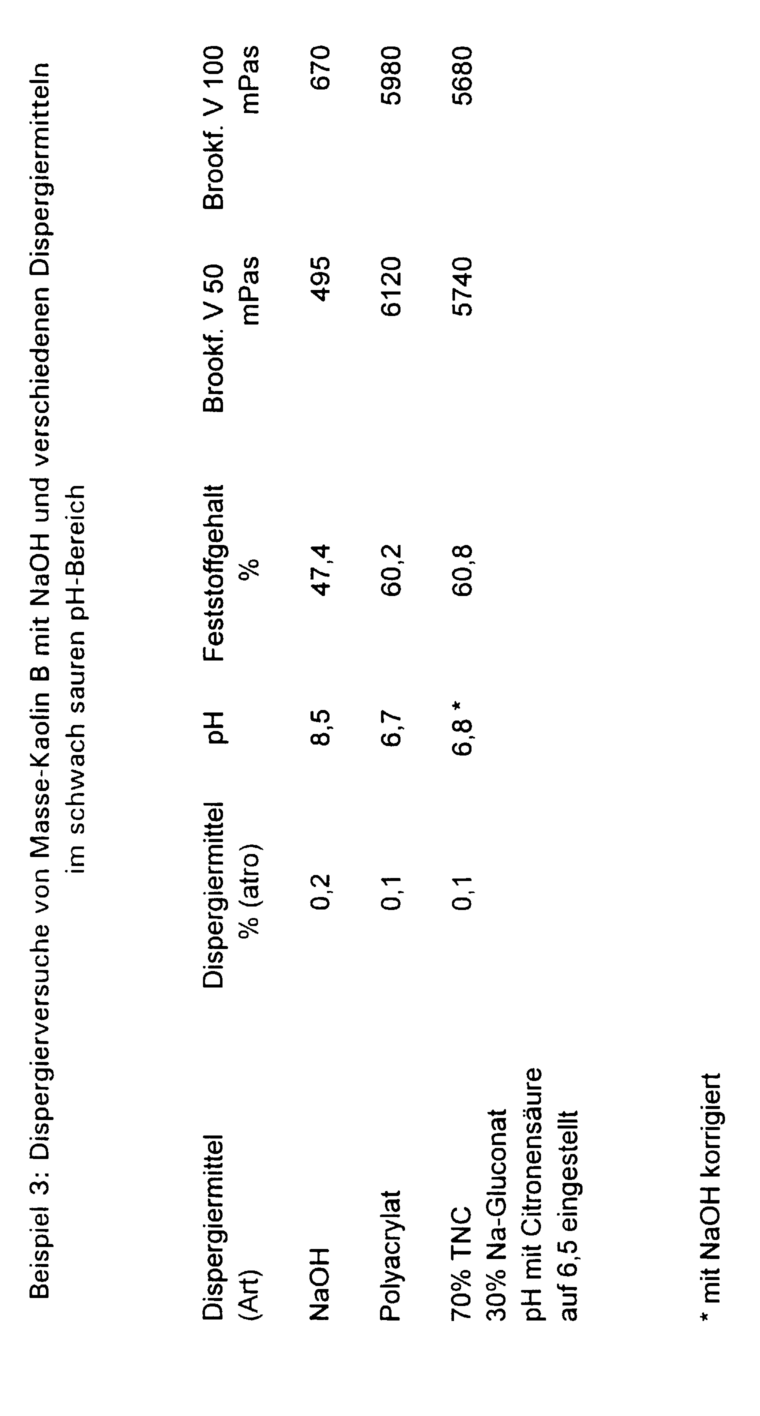

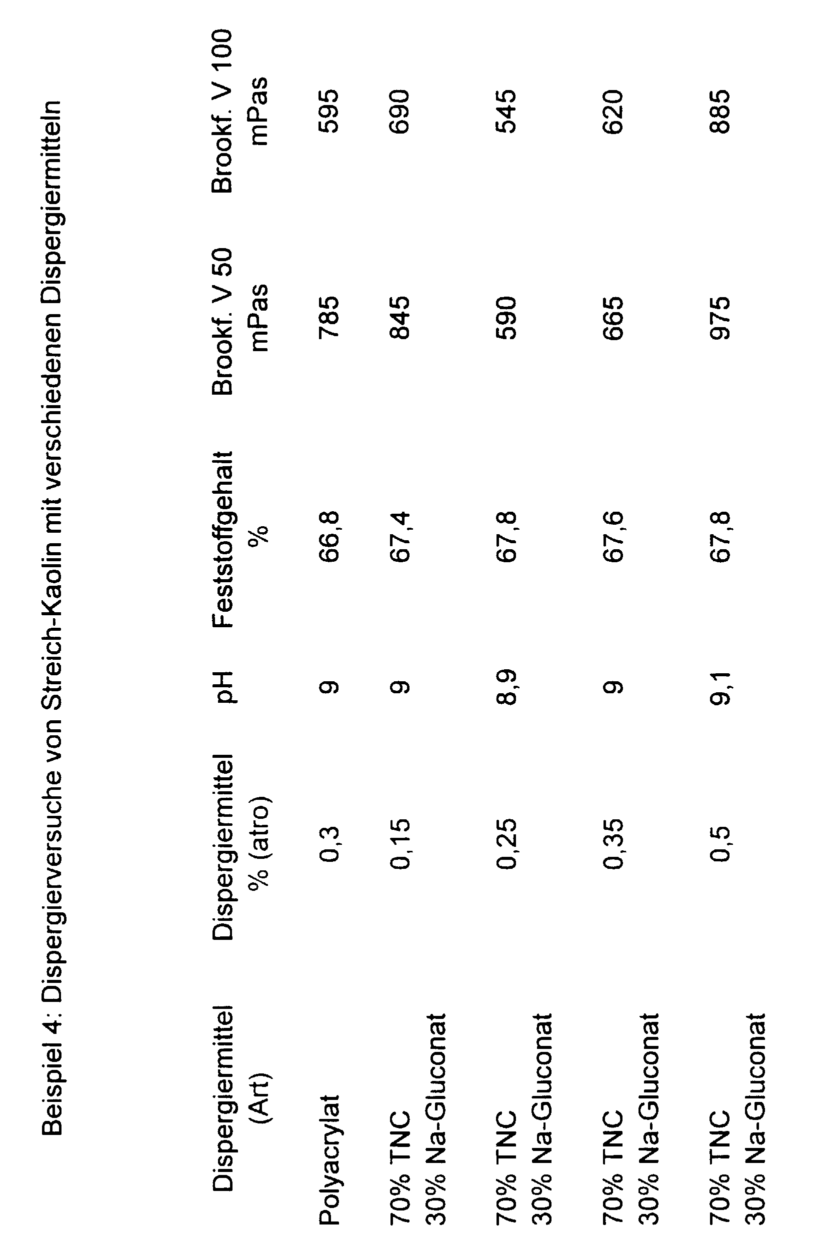

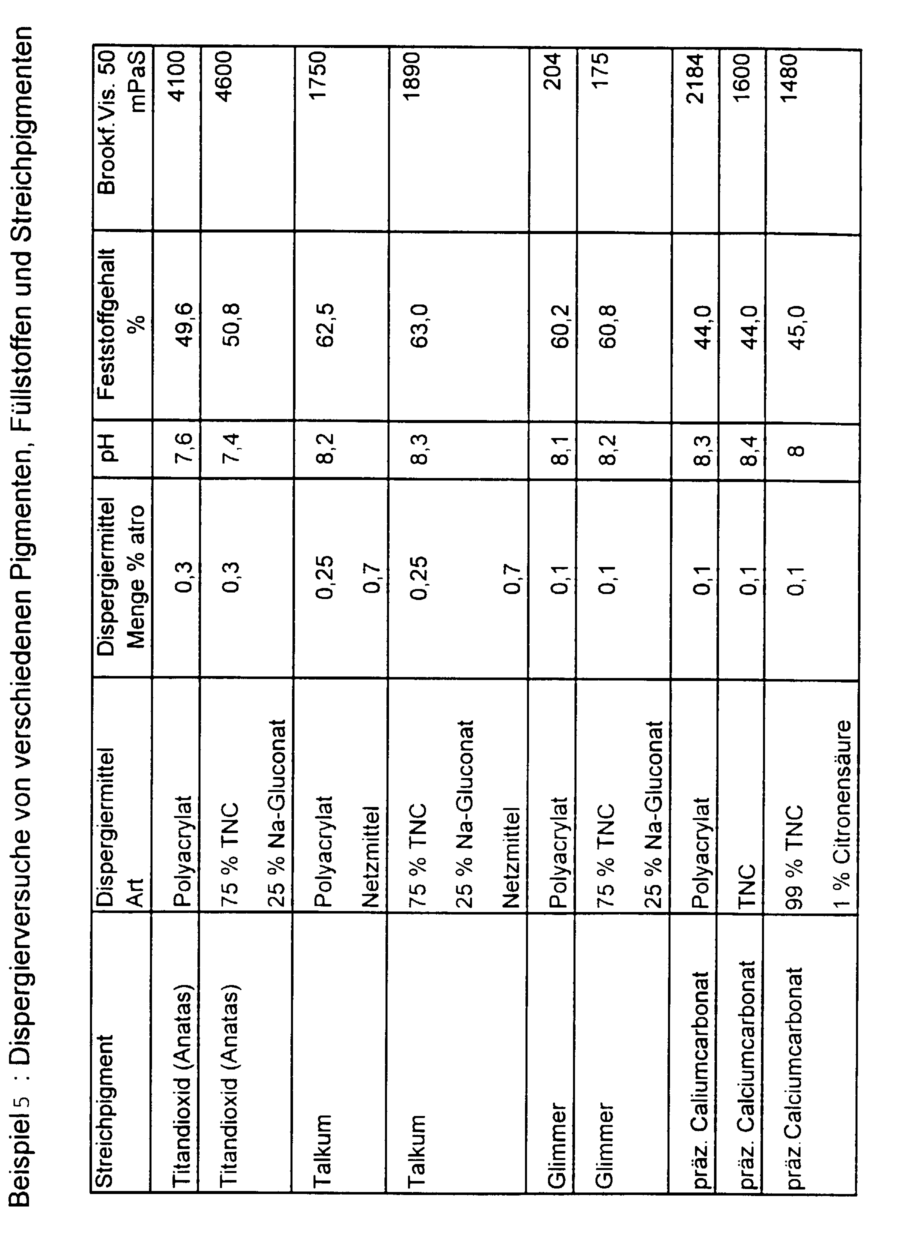

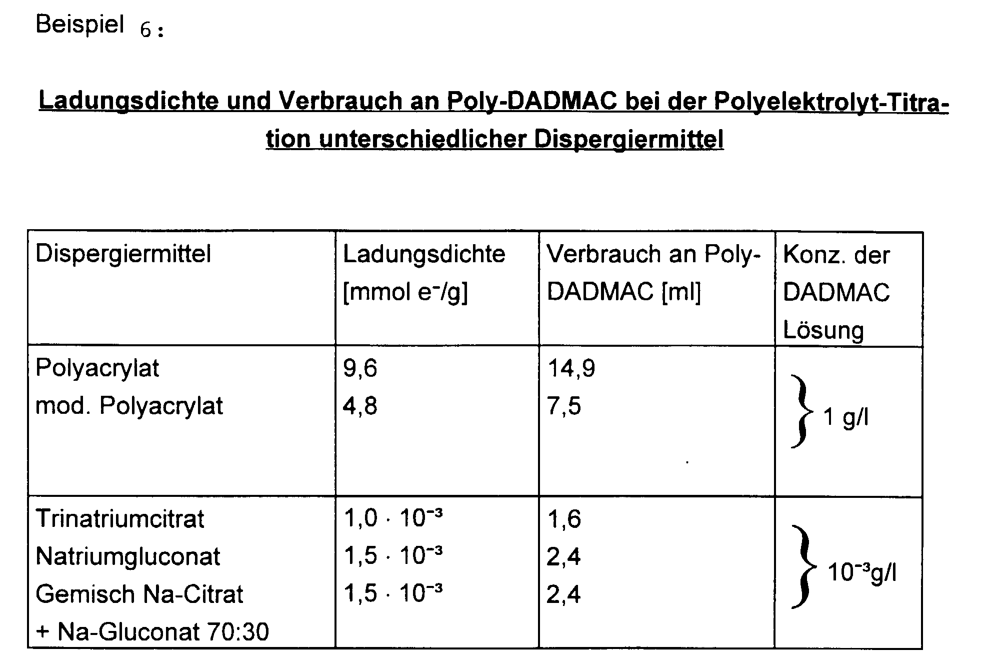

| Beispiel 7: | | | |
|---|---|---|---|
| **Zeta-Potential-Messung von Füllstoffen und Pigmenten mit verschiedenen Dispergiermitteln** | | | |
| Pigment | Dispergiermittel (Art) | Dispergiermittelmenge (% atro) | ZP (mV) |
| Masse Kaolin | Polyacrylat | 0,1 | - 78,5 |
| Masse Kaolin | 75 % TNC 25 % Gluconat Citronensäure pH 6,4 | 0,1 | - 59,0 |
| TiO₂ (Anatas) | Polyacrylat | 0,3 | - 72,0 |
| TiO₂ (Anatas) | 70 % TNC 30 % Gluconat | 0,3 | - 47,5 |

## Patentansprüche

1. Dispergier- und Mahlhilfsmittel für anorganische Füllstoffe und/oder Pigmente , **dadurch gekennzeichnet,** daß es aus einer Mischung von Citronensäure und Gluconsäure sowie ihren Alkalisalzen besteht, wobei das Gemisch in der Füllstoffdispersion einen pH-Wert von 4,0 bis 10,5 aufweist, und Citrat und Gluconat in einem Verhältnis von 1 : 20 bis 20 : 1 vorliegen.

2. Dispergiermittel gemäß Anspruch 1, **dadurch gekennzeichnet,** daß zusätzlich noch eine oder mehrere Hydroxycarbonsäuren mit 3 bis 10 C-Atomen , welche 1 bis 6 Carbonsäuregruppen und 1 bis 5 OH-Gruppen enthalten, enthalten sind.

3. Dispergiermittel gemäß Anspruch 2, **dadurch gekennzeichnet**, daß die Hydroxycarbonsäure Weinsäure, Äpfelsäure, Glucarsäure, Glucuronsäure oder ihre Natrium- und/oder Kaliumsalze ist.

4. Dispergiermittel gemäß Anspruch 1, **dadurch gekennzeichnet**, daß durch Zugabe von Alkalihydroxid oder -carbonat ein pH-Wert der Dispersion von 8,3 bis 9,0 oder durch Zugabe der entsprechenden Säuren auf 6,0 bis 6,8 eingestellt wird.

5. Dispergierhilfsmittel gemäß einem der Ansprüche 1 bis 4, **dadurch gekennzeichnet**, daß Citrat und Gluconat im Verhältnis von 1 : 2 bis 5 : 1, eingesetzt werden.

6. Dispergiermittel gemäß einem der Ansprüche 1 bis 5, **dadurch gekenn-zeichnet**, daß die Füllstoffe und/oder Pigmente anorganische Füllstoffe und/oder Pigmente für die Papierherstellung und -veredelung darstellen.

7. Verwendung von Dispergiermitteln gemäß einem der Ansprüche 1 bis 6, **dadurch gekennzeichnet,** daß zur Herstellung von Dispersionen mit einem Feststoffgehalt von >30 %, vorzugsweise >50 %, Dispergiermittelmengen von 0,05 bis 2 %, vorzugsweise 0,1 bis 1 %, bezogen auf die Feststoffe, verwendet werden.

8. Verwendung von Dispergiermitteln gemäß Anspruch 7, **dadurch gekennzeichnet**, daß die Dispersionen eine Viskosität von unter 3000, vorzugsweise 150 - 1000 mPas aufweisen.

9. Verwendung von Dispergiermitteln gemäß Anspruch 7 oder 8 zur Lack-, Farben-, Keramik-, Gummi-, Kunststoff- und insbesondere der Papierherstellung und -veredelung.

## Claims

1. A dispersion agent and grinding aid for inorganic fillers and/or pigments characterised in that it comprises a mixture of citric acids and gluconic acid and their alkali salts, whereby the mixture has a pH value of 4.0 to 10.5 in the filler dispersion and the ratio of citrate to gluconate is in the range of 1 : 20 to 20 : 1.

2. The dispersion agent according to claim 1, wherein it contains additionally one or more hydroxycarboxylic acids with 3 to 10 carbon atoms, containing 1 to 6 carboxy groups and 1 to 5 hydroxy groups.

3. The dispersion agent according to claim 2, wherein the hydroxycarboxylic acid is tartaric acid, malic acid, glucaric acid, gluconic acid or the sodium or potassium salts thereof.

4. The dispersion agents according to claim 1, wherein the pH value of the dispersion is adjusted to 8.3 - 9.0 by adding alkali hydroxide or carbonate or to 6.0 - 6.3 by adding the respective acids.

5. The dispersion agent according to one of claims 1 to 4, wherein citrate and gluconate are used in ratios of from 1 : 2 to 5 : 1.

6. The dispersion agent according to one of claims 1 to 5, wherein said fillers and/or pigments are inorganic fillers and/or pigments for the production and refining of paper.

7. The use of the dispersion agent according to one of claims 1 to 6 for the production of dispersions having a solid matter content greater than 30 %, preferably greater than 50 %, wherein the quantity of dispersion agent used is between 0.05 - 2.0 wt%, preferably between 0.1 - 1 wt% of said solid matter content.

8. The use of the dispersion agent according to claim 7, wherein said dispersions show a viscosity of under 3000 mPas, preferably of from 150 - 1000 mPas.

9. The use of the dispersion agent according to claim 7 or 8 for the production of varnishes, paints, ceramics, rubber, plastics, and especially for the production and refining of paper.

## Revendications

1. Auxiliaire de dispersion et de broyage pour charges et/ou pigments inorganiques, caractérisé en ce qu'il est constitué d'un mélange d'acide citrique et d'acide gluconique, de même que de leurs sels alcalins, le mélange dans la dispersion de charges présentant un pH de 4,0 à 10,5, et le citrate et le gluconate étant présents en un rapport de 1 : 20 à 20 : 1.

2. Agent de dispersion selon la revendication 1, caractérisé en ce qu'en outre un ou plusieurs acides hydroxycarboxyliques avec 3 à 10 atomes de carbone, qui contiennent 1 à 6 groupes d'acide carboxylique et 1 à 5 groupes OH, y sont contenus.

3. Agent de dispersion selon la revendication 2, caractérisé en ce que l'acide hydroxycarboxylique est l'acide tartrique, l'acide malique, l'acide glucarique, l'acide glucuronique ou leurs sels de sodium et/ou de potassium.

4. Agent de dispersion selon la revendication 1, caractérisé en ce qu'on règle, par addition d'hydroxyde alcalin ou de carbonate alcalin, un pH de la dispersion de 8,3 à 9,0 ou, par addition des acides correspondants, un pH de 6,0 à 6,8.

5. Auxiliaire de dispersion selon l'une des revendications 1 à 4, caractérisé en ce que le citrate et le gluconate sont utilisés en un rapport de 1 : 2 à 5 : 1.

6. Agent de dispersion selon l'une des revendications 1 à 5, caractérisé en ce que les charges et/ou les pigments représentent des charges et/ou des pigments inorganiques pour la fabrication et le finissage du papier.

7. Utilisation d'agents de dispersion selon l'une des revendications 1 à 6, caractérisée en ce qu'on utilise, pour la fabrication de dispersions avec une teneur en matières solides supérieure à 30 %, de préférence supérieure à 50 %, des quantités d'agent de dispersion de 0,05 à 2 %, de préférence de 0,1 à 1 %, par rapport aux matières solides.

8. Utilisation d'agents de dispersion selon la revendication 7, caractérisée en ce que les dispersions présentent une viscosité inférieure à 3.000, de préférence de 150 à 1.000 mPas.

9. Utilisation d'agents de dispersion selon la revendication 7 ou 8 pour la fabrication et le finissage de vernis, de peintures, de céramiques, de caoutchoucs, de matières synthétiques et en particulier de papier.
